Europäisches Patentamt

European Patent Office

Office européen des brevets

(11) Publication number: **0 196 340**
**B1**

# EUROPEAN PATENT SPECIFICATION

(45) Date of publication of patent specification: **29.06.88**

(21) Application number: **85103746.5**

(22) Date of filing: **28.03.85**

(51) Int. Cl.⁴: **C 12 F 3/08**, A 61 K 7/06, A 61 K 7/48

(54) Modified alcohol useful for fragrance or cosmetics.

(43) Date of publication of application:
08.10.86 Bulletin 86/41

(45) Publication of the grant of the patent:
29.06.88 Bulletin 88/26

(84) Designated Contracting States:
CH DE FR LI LU

(56) References cited:
FR-A- 678 319
GB-A-2 060 378

SEIFEN-OLE-FETTE-WACHSE, vol. 111, no. 1,
17th January 1985, pages 23-25, Augsburg, DE;
B. STELLMACH: "Herstellung und
Standardisierung von Pflanzenextrakten"

CHEMICAL ABSTRACTS, vol. 81, no. 9, 28th
October 1974, page 348, no. 103183, Columbus,
Ohio, US; N.I. RAZUVAEV: "Current methods
for processing marc abd wine drugs", &
LOZAR. VINAR. 1973, 22(8), 33-40

(73) Proprietor: Takasago Perfumery Co., Ltd.
3-28-9 Takanawa
Minato-ku Tokyo 108 (JP)

(72) Inventor: Hosoda, Bunichiro
1-17-8 Jiyugaoka Meguro-ku
Tokyo 152 (JP)
Inventor: Moroe, Michio
2-28-9 Shimorenjaku
Mitaka-shi Tokoyo 181 (JP)

(74) Representative: Bühling, Gerhard, Dipl.-Chem.
et al
Patentanwaltsbüro Tiedtke-Bühling-Kinne
Grupe-Pellmann-Grams-Struif Bavariaring 4
D-8000 München 2 (DE)

Courier Press, Leamington Spa, England.

**Description**

BACKGROUND OF THE INVENTION

(a) Technical Field of the Invention

This invention relates to a modified alcohol useful as a raw material for fragrance or cosmetics such as perfume, eau de Cologne and other cosmetics, and more particularly to a modified alcohol for fragrance or cosmetics, which is denatured to eliminate pungent odor inherent to ethanol.

(b) Description of the Prior Art

Generally, ethanol is employed as a main component for fragrance or cosmetics such as perfume, eau de Cologne, hair cosmetics and other cosmetics in order to invest such fragrance or cosmetics with properties such as astringency, cleansing effects, sterilizing property, solubility, an improved volatility and an excitement effect.

Such an ethanol (or modified ethanol) as useful for fragrance or cosmetics is generally produced by a fermentation method employing as a raw material molasses, starch, a waste fluid from a pulp plant and the like. Consequently, such an ethanol generally has a peculiar odor and a burning taste according to the differences in production region and in raw material.

Recently, it has become possible to produce a substantially odorless ethanol by a systhesis. The quality of the ethanol produced by the synthesis however is still unsatisfactory.

A modified alcohol, which is denatured to remove an pungent odor of ethanol by the distillation of a fruit wine or by utilizing a residue left from the production of a essential oil of jasmine, rose or lavender which is cultivated in the Mediterranean region, is also utilized as a solvent in a perfume or eau de Cologne.

However, such a modified alcohol production is limited in quantity at low cost performance.

SUMMARY OF THE INVENTION

The main object of this invention is to provide a modified alcohol for fragrance or cosmetics, which is denatured to substantially eliminate pungent odor peculiar to an ordinary ethanol, and which has a good conformity with a perfume oil.

Another object of this invention is to provide a modified alcohol, which is suited for a high quality fragrance or cosmetics and can be produced in large quantity and at high cost performance.

These objects have been accomplished according to this invention by contacting ethanol with a fermented grape residue produced in a wine manufacturing process, thereby to produce an extracted liquid, and refining the extracted liquid.

DETAILED DESCRIPTION OF THE PREFERRED EMBODIMENTS

Fermentation residue of grape fruit (hereinafter referred to as "wine residue") obtained in the manufacturing process of grape wine should be understood in this invention to mean the residue of grape fruit which are usually produced in the conventional grape wine manufacturing process. Such residue can be obtained as a result of a grape fermentation process comprising the steps of removing stalks of grape fruits by using a crushing and stalks-removing machines, fermenting the resulted grape fruit juice after adding yeast and other additives, separating a fermented product to leave a residue, and treating the residue in a filter press to filtering off a liquid material thereby leaving the wine residue. Generally, wine residue obtained in red wine-manufacturing process can be conveniently employed, but other kinds of wine residue could be employed.

There is no limitation with respect to ethanol to be employed in this invention. However, it is preferable to employ an industrial alcohol which conforms to JIS regulations, irrespective of its origin, i.e. a synthetic alcohol or a fermented alcohol.

Modified alcohol for fragrance or cosmetics according to this invention can be manufactured by the following process.

One part by weight of wine residue is mixed with one to 100 parts by weight of an industrial ethanol. The resulted mixture is then agitated for one to 10 hours at a temperature ranging from room temperature to 80°C, or, in another method, allowed to stand without being agitated at room temperature for 2 to 3 days.

Then, the wine residue are separated through a filteration, and resulted filtrate is distilled under a reduced pressure of 53,32 to 66,65 mbar (40 to 50 mmHg) to obtain a distillate having a boiling point of 20 to 40°C. This distillation process may be carried out at a reduced pressure ranging from 13,33 to 666,5 mbar (10 to 500 mmHg) in order to obtain a distillate having a boiling point substantially corresponding to the range mentioned above.

In another method of manufacturing modified alcohol for fragrance or cosmetics, wine residue are removed of their seeds and then dried out to contain 1 to 3% by weight of water and 1 to 2% by weight of alcohol. The resulted granular residue are filled in a column, and then industrial ethanol is dripped at the top of the column, thereby contacting the ethanol with the granular residue. The ethanol passing down through the granular residue is withdrawn at the bottom of the column and then distilled under a reduced pressure in the same manner as mentioned above.

The alcohol thus obtained is free from a pungent odor which is peculiar to the conventional ethanol and, in place of that pungent odor, is invested with a pleasant fermentation odor originated from the wine

2

residue. Accordingly, the alcohol thus obtained is not only extremely suited as a solvent for fragrance or cosmetics, but also has a good conformity with a perfume oil.

This invention is also advantageous in view of an effective utilization of wine residue which have so far been deemed only as a waste material in the grape wine manufacturing industry. Accordingly, this invention would be of a great benefit to the industry in the stand-point of the reutilization of resources.

Since the wine residue from grape wine manufacturing process are available in cheap and large quantity, the modified alcohol for fragrance or cosmetics can be manufactured at lower cost and in large quantity.

The modified alcohol for fragrance or cosmetics of this invention can be utilized as a component for a make-up liquid, a hair-tonic, a hair-liquid, a set-lotion, other ethanol-containing cosmetics, in addition to a perfume and eau de Cologne.

This invention will be further explained with reference to the following examples.

In the following examples, a perfume oil of rose type is employed. Because this type of perfume oil is most sensitive to the ethanolic odor as compared to other perfume oils and therefore suited in the assessment of the properties of the modified alcohol of this invention.

Example 1

1000 g of an industrial ethanol was added to 100 g of a wine residue to obtain a mixture. The mixture was then stirred for two hours at room temperature and then subjected to filtration using a glass filter (G3) to obtain 1060 g of a purple filtrate containing ethanol. This purple filtrate was then distilled under a reduced pressure of 50 mmHg to obtain 720 g of a distillate having a boiling point of 20 to 40°C into an ice-cooled receiver. The distillate had a specific gravity of 0.8153 (15°C) and was found to have a high purity which conforms to the regulation of the raw cosmetics material standards as Japanese Standards of Cosmetic Ingredients.

The purity tests were conducted on the following items of Japanese Standards of Cosmetic Ingredients:

(1) *Clarity and Color*

When 10 ml of the modified alcohol for fragrance or cosmetics as obtained in the Example 1 (hereinafter referred to as "the product of this invention") were mixed with 30 ml of water and allowed to stand for 30 minutes at 8 to 10°C, the alcohol solution maintained the original clearness thereof.

(2) *Acid and Alkali*

20 ml of distilled water were added to 20 ml of the product of this invention, and then three drops of phenolphthalein reagent were added to the resulted mixture. However, the color of the mixture remained unchanged. When 0.1 ml of 0.1N sodium hydroxide was further added to the mixture, the mixture indicated a red color.

(3) *Cloride*

No change in outlook of the product of this invention was recognized, even when 10 ml of the product of this invention were added with two drops of a silver nitrate test solution and then allowed to stand for 5 minutes.

(4) *Heavy Metals*

An analysis of the product of this invention with respect to heavy metals was conducted as follows. First, 2 ml of dilute acetic acid and water were added to 30 ml of the product of this invention to obtain 50 ml of a mixed solution and then this mixed solution was used for the analysis as a test solution. As a result, the product of this invention was found to contain 0.9 ppm of heavy metals.

(5) *Fusel Oil and other similar impurities*

To 10 ml of the product of this invention were added 5 ml of water and 1 ml of glycerine. This mixed solution was then dripped onto a filter paper and subsequently allow to stand at room temperature to evaporate the solution. As a result, no foreign odor was recognized on the filter paper.

Further, when 5 ml of the product of this invention was cautiously stratified in a test tube filled with 5 ml of sulfuric acid. The border surface of two layers does not indicate a red color.

(6) *Aldehyde or other reducing substances*

When 0.3 ml of 0.1N potassium permanganate was added to 10 ml of the product of this invention at 15°C and then allowed to stand for 20 minutes under the same temperature as before, the red color of the potassium permanganate was not vanished.

When 5 ml of sodium hydroxide test solution were added to 10 ml of the product of this invention, and then allowed to stand for 5 minutes, the solution did not indicate a yellow color.

3

(7) *Methanol and Acetone*

The product of this invention had conformed to the test accordance with methanol and acetone determination.

(8) *Residue of Evaporation*

When 40 ml of the product of this invention were evaporated over a water bath and the resulted residual portion were dried at 105°C for one hour to obtain 0.8 mg of residual materials.

(9) *Odor*

The product of this invention was a colorless transparent liquid and had a sweet smelling fermentation odor.

## Example 2

To 200 g of wine residue were added 2000 g of industrial ethanol and the resulted mixture was refluxed under boiling while being agitated for one hour at a temperature of 70 to 80°C. Subsequently the resulted product was cooled and filtered to obtain a purple filtrate containing ethanol. This filtrate was treated in the same manner as in Example 1 to obtain 1420 g of fraction. The quality of the fraction was found to be almost the same as in that of the Example 1.

## Example 3

To 100 g of wine residue were added 1000 g of industrial ethanol. The resulted mixture was homogeneously agitated and allowed to stand for two full days at about 20°C. Then, this mixture was filtered and the obtained filtrate was treated in the same manner as in Example 1 to obtain 715 g of fraction. This fraction was found to have almost the same quality as that of Example 1.

## Example 4

1 kg of wine residue was minced with a meat-mincing machine and then extruded to obtain cord-like wine residue. The cord-like wine residue were dried under the sun for two days to obtain granules containing 2.2% of water (Karl Fischer Method) and 1.4% of alcohol (Evaporated Water-Specific Gravity Measuring Method). The granules were filled in a glass cylinder having a diameter of 5 cm and a constricted bottom portion on which a G3 filter is mounted. The 5 kg of industrial ethanol were poured in the glass cylinder at the rate of 10 to 50 ml per minute to obtain 3.7 kg of colored filtrate containing ethanol as an effluent from the bottom of the glass cylinder. This filtrate was treated in the same manner as in Example 1 to obtain 2.59 kg of fraction. This fraction was found to have almost the same quality as that of the Example 1.

## Example 5

Two different perfumes were manufactured according to the following prescription and the resulted perfumes were subjected to comparative sensitivity tests.

Prescription:

| | |
|---|---|
| Rose type perfume oil BF—1473 (manufactured by Takasago Perfumery Co. Ltd.) | 20% (by weight) |
| Ethanol* | 80% (by weight) |

*Ethanol A: Untreated industrial alcohol (standardized article according to Japanese Standards of Cosmetic Ingredients).

Ethanol B: Alcohol for fragrance or cosmetics produced in Example 1 according to this invention.

Ten experienced panelists for sensitivity tests (hereinafter referred to as panelists) took part in odor test for assessing the pungent odor of ethanol in four stages method. As a result, a perfume manufactured by employing the products of this invention was found as shown Table 1 to have little pungent odor peculiar to ethanol and a good conformity with the perfume oil.

4

**0 196 340**

Table 1 (Results of Test)

| Panelists | Ethanol A (untreated) | Ethanol B (this invention) |
|---|---|---|
| Panelist 1 | + + | - |
| Panelist 2 | + | - |
| Panelist 3 | + | - - |
| Panelist 4 | + + | - |
| Panelist 5 | + | - |
| Panelist 6 | + | - |
| Panelist 7 | + + | - |
| Panelist 8 | + + | - - |
| Panelist 9 | + | - |
| Panelist 10 | + | - |

This assessment of the pungent odor of ethanol was conducted on the basis of following four stages.

"++": The pungent odor was strongly felt.

"+": The pungent odor was slightly felt.

"−": The pungent odor was very slightly felt.

"−−": The pungent odor was not felt at all.

Example 6

Two different eau de Cologne were manufactured according to the following prescription and the resulted eau de Cologne were subjected to comparative sensitivity tests.

Prescription:

Rose type perfume oil BF—3002
(manufactured by Takasago Perfumery Co. Ltd.)    5% (by weight)

Ethanol*                                          95% (by weight)

*Ethanol A: Untreated industrial alcohol (standardized article according to Japanese Standards of Cosmetic Ingredients).

Ethanol B: Alcohol for fragrance or cosmetics produced in Example 1 according to this invention.

Eight experienced panelists (hereinafter referred to as panelists) took part in sensitivity tests for assessing the pungent odor of ethanol and the conformity of ethanols A and B with the perfume oil. As a result, an eau de Cologne manufactured by employing the products of this invention was found as shown in Table 2 to have little pungent odor peculiar to ethanol and a good conformity with the perfume oil.

The assessment of the pungent odor of ethanol was conducted in the same manner as in Example 5.

5

Table 2

| Panelists | Ethanol A | | Ethanol B | |
|---|---|---|---|---|
| | Pungent Odor | Conformity | Pungent Odor | Conformity |
| Panelist 1 | + | * * | - | * * * |
| Panelist 2 | + | * * | - | * * |
| Panelist 3 | + | * * | - | * * * |
| Panelist 4 | + + | * | - - | * * * |
| Panelist 5 | + | * * | - | * * * |
| Panelist 6 | + | * * | - | * * * |
| Panelist 7 | + | * * | - - | * * |
| Panelist 8 | + + | * | - | * * * |

The assessment of conformity with the perfume oil was conducted in the following three stages method.

"***": Conformity with the perfume oil is very excellent.

"**": Conformity with the perfume oil is good.

"*": Conformity with the perfume oil is somewhat poor.

## Claims

1. Modified alcohol useful for fragrance or cosmetics, which is characterized by including a modified ethanol which is distilled from an extracted liquid obtained by contacting ethanol with a fermented grape residue produced in a wine manufacturing process.

2. Modified alcohol according to claim 1, characterized in that said residue is produced by first adding yeast to a grape juice to cause fermentation of said juice and then filtering off a liquid portion thereby leaving a solid fraction of the grape residue.

3. Modified alcohol according to claim 1, characterized in that said residue is produced by first adding yeast to a grape juice, removing a liquid portion from the fermented juice thereby leaving a fermented solid residue, removing seeds from the solid residue, and drying the solid residue.

4. Modified alcohol according to claim 1, characterized in that said ethanol consists of distillates having boiling points of 20 to 40°C at a reduced pressure ranging from 53,32 to 66,65 mbar (40 to 50 mm Hg).

## Patentansprüche

1. Modifizierter Alkohol, der für Duftstoffe oder Kosmetika brauchbar ist und dadurch gekennzeichnet ist, daß er ein modifiziertes Ethanol enthält, das aus einer Extraktionsflüssigkeit herausdestilliert wird, die erhalten wird, indem Ethanol mit einem vorgorenen, in einem Weinherstellungsverfahren erzeugten Traubenrückstand in Berührung gebracht wird.

2. Modifizierter Alkohol nach Anspruch 1, dadurch gekennzeichnet, daß der erwähnte Rückstand erzeugt wird, indem zuerst zu einem Traubensaft Hefe hinzugegeben wird, um eine Vergärung des erwähnten Saftes zu bewirken, und dann ein flüssiger Anteil abfiltriert wird, wodurch eine feste Fraktion des Traubenrückstands zurückgelassen wird.

3. Modifizierter Alkohol nach Anspruch 1, dadurch gekennzeichnet, daß der erwähnte Rückstand erzeugt wird, indem zuerst zu einem Traubensaft Hefe hinzugegeben wird, aus dem vergorenen Saft ein flüssiger Anteil entfernt wird, wodurch ein vergorener fester Rückstand zurückgelassen wird, aus dem festen Rückstand Samen bzw. Kerne entfernt werden und der feste Rückstand getrocknet wird.

4. Modifizierter Alkohol nach Anspruch 1, dadurch gekennzeichnet, daß das erwähnte Ethanol aus Destillaten besteht, die bei einem verminderten Druck von 53,32 bis 66,65 mbar (40 bis 50 mm Hg) Siedepunkte von 20 bis 40°C haben.

**Revendications**

1. Alcool modifié utilisé pour des produits de parfumerie ou de beauté, caractérisé en ce qu'il comporte un éthanol modifié, qui est distillé à partir d'un liquide extrait obtenu en mettant en contact de l'éthanol avec un résidu de raison fermenté produit dans un processus de fabrication du vin.

2. Alcool modifié selon la revendication 1, caractérisé en ce que ce résidu est produit en ajoutant d'abord de la levure à un jus de raisin pour provoquer la fermentation et en en extrayant ensuite par filtration une portion liquide, laissant ainsi une portion solide du résidu de raisin.

3. Alcool modifié selon la revendication 1, caractérisé en ce que ce résidu est produit en ajoutant d'abord de la levure à un jus de raisin, en enlevant une portion liquide du jus fermenté, laissant ainsi un résidu solide fermenté, en enlevant les pépins du résidu solide, et en séchant le résidu solide.

4. Alcool modifié selon la revendication 1, caractérisé en ce que cet éthanol est constitué par des distillats ayant des points d'ébullition compris entre 20 et 40°C à une pression réduite comprise entre 53,32 et 66,65 mbar.